(19) Europäisches Patentamt
European Patent Office
Office européen des brevets

(11) Publication number: 0 247 845 A1

# (12) EUROPEAN PATENT APPLICATION

(21) Application number: 87304683.3

(22) Date of filing: 27.05.87

(51) Int. Cl.4: C 12 Q 1/28
G 01 N 33/52
// C09B23/00

(30) Priority: 30.05.86 US 868474

(43) Date of publication of application:
02.12.87 Bulletin 87/49

(84) Designated Contracting States: CH DE FR GB LI

(71) Applicant: EASTMAN KODAK COMPANY
Patent Department 343 State Street
Rochester New York 14650 (US)

(72) Inventor: Norton, Gary Edward c/o EASTMAN KODAK COMPANY
Patent Deparment 343 State Street
Rochester New York 14650 (US)

(74) Representative: Baron, Paul Alexander Clifford et al
Kodak Limited Patent Department Headstone Drive
Harrow Middlesex HA1 4TY (GB)

## (54) Use of methine dyes for the determination of hydrogen peroxide or analytes producing same.

(57) Methine dyes are useful in the determination of hydrogen peroxide, or of analytes which can react to produce hydrogen peroxide, in the presence of a peroxidative substance. These dyes are also useful for the determination of peroxidase or other peroxidative substances. Such determinations can be carried out in solution or with dry analytical elements.

EP 0 247 845 A1

## Description

# USE OF METHINE DYES FOR THE DETERMINATION OF HYDROGEN PEROXIDE OR ANALYTES PRODUCING SAME

This invention relates to the use of methine dyes to detect hydrogen peroxide or other analytes which react to produce hydrogen peroxide. It also relates to the use of methine dyes to determine peroxidase or other peroxidative substances. This invention is particularly useful for the assay of biological fluids in clinical chemistry.

The detection and quantitative determination of hydrogen peroxide and compounds yielding hydrogen peroxide as a result of chemical or enzymatic reactions are of importance in may areas. For example, they are important in the detection of hydrogen peroxide produced in the enzymatic assay of chemical or biological substances (sometimes called analytes) such as glucose, cholesterol, uric acid, triglycerides or creatine kinase. The quantity of analyte present in a specimen sample is determinable from the amount of hydrogen peroxide produced and detected.

Known compositions for detecting or quantifying hydrogen peroxide in such assays generally comprise a substance having peroxidative activity, for example peroxidase, and a material which undergoes a detectable change (for example a color change) in the presence of hydrogen peroxide and the peroxidative substance. Various materials which undergo such a detectable change include monoamines, diamines, phenols, leuco dyes and other known dyes or dye-providing materials. Dye-providing materials also useful in such assays include triarylimidazoles as described, for example, in U. S. Patent 4,089,747.

However, it has been observed that many dye-providing materials used to detect hydrogen peroxide readily oxidize in aqueous solutions containing a substance having peroxidative activity or in air. This instability renders them unsuitable for analytical determinations, for example dry assays where the analytical composition is stored for a period of time prior to use.

Although other dye-providing materials have improved stability and are, in general, useful as indicators for hydrogen peroxide determination, there are instances when the concentration of hydrogen peroxide to be analyzed is too low to produce sufficient detectable color from such indicators. Where high dilution of the test sample is required, such indicators may also provide insufficient detectable color.

Hence, there is a continuing need in the art for a reliable means for detecting low concentrations of hydrogen peroxide or of analytes which react to produce same.

The problems noted above for determining hydrogen peroxide or an analyte which is capable of reacting to produce hydrogen peroxide have been solved according to this invention with an analytical composition comprising a peroxidative substance, the composition characterized in that it also comprises a methine dye capable of being oxidized by hydrogen peroxide in the presence of the peroxidative substance.

The present invention also provides an analytical element for the determination of hydrogen peroxide or an analyte which is capable of reacting to produce hydrogen peroxide comprising an absorbent carrier material, and containing a peroxidative substance, the element characterized in that it comprises a methine dye capable of being oxidized by hydrogen peroxide in the presence of the peroxidative substance.

There is also provided a method for the determination of hydrogen peroxide or an analyte which will react to produce hydrogen peroxide comprises the steps of:

A. in the presence of a peroxidative substance, contacting a sample of a liquid suspected of containing hydrogen peroxide or an analyte which will react to produce hydrogen peroxide with a methine dye capable of being oxidized by hydrogen peroxide to produce a detectable change, and

B. determining the detectable change resulting from the presence of hydrogen peroxide or the analyte.

Still further, a method for the determination of a peroxidative substance comprises:

A. in the presence of hydrogen peroxide or an interactive composition which is capable of reacting to produce hydrogen peroxide, contacting a sample of a liquid suspected of containing a peroxidative substance with a methine dye capable of being oxidized by hydrogen peroxide in the presence of the peroxidative substance to produce a detectable change, and

B. determining the detectable change resulting from the presence of the peroxidative substance.

The present invention provides a means for detecting low levels of hydrogen peroxide or analytes which will react to produce hydrogen peroxide. This assay is rapid and produces a significant detectable change even in the presence of low concentrations of hydrogen peroxide. The methine dyes are not affected by reductive substances often found in biological fluids, for example ascorbate. Further, the methine dyes are readily adapted to dry analytical elements and do not necessitate the use of organic coating solvents due to their solubility in aqueous coating solutions.

The FIGURE is a graphical plot of reflection density ($D_R$) versus time in minutes for the dry analytical assay of hydrogen peroxide described in Example 4 below.

Any methine dye which is capable of being oxidized by hydrogen peroxide in the presence of a peroxidative substance can be used in the practice of this invention. A simple test can be carried out to determine if a given methine dye is useful in the present invention. A dye ($10^{-6}$ molar) is mixed with hydrogen peroxide ($10^{-3}$ molar) and peroxidase ($10^{-9}$ molar) in a buffered solution (pH 7) at 25°C. The spectral absorption of the solution is then measured. If, after 5 minutes, a change in the spectral absorption is detectable, the methine dye can be used in the practice of this invention.

Preferably, the methine dyes have an oxidation potential in the range of from 0.20 to 1.5 volts. More preferably, the oxidation potential is from 0.5 to 1.0 volts. The oxidation potentials of many methine dyes are available in the literature. However, they can also be readily measured by standard AC voltammetry as described, for example, by Bond and Smith in Anal. Chem., 46, 1946 (1974).

Generally, the methine dyes useful in the practice of this invention contain substituted or unsubstituted methine linkages connecting groups which are independently substituted or unsubstituted heterocyclic, carbocyclic, acyclic, aromatic or amino groups. Preferably, such dyes are represented by the structure:

$(\text{A-METH-B}\ )_m(\ \text{METH-D})_n$

wherein A, B and D are independently substituted or unsubstituted heterocyclic, carbocyclic, acyclic, aromatic or amino groups, m is 1 and n is 0 or 1, and preferably n is 0. The A, B and D groups can individually have an ionic or neutral charge in tautomeric forms. METH is the substituted or unsubstituted methine linkage described above. More particularly, METH can be represented by the structure:

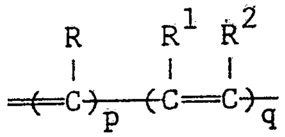

wherein R, $R^1$ and $R^2$ are independently hydrogen, substituted or unsubstituted alkyl (preferably of 1 to 4 carbon atoms, for example, methyl, ethyl, propyl or isopropyl), aryl (preferably of 6 to 12 carbon atoms, for example, phenyl, xylyl or naphthyl), or any two of R, $R^1$ or $R^2$ can represent the atoms necessary to complete an alkylene or heterocyclic bridge, p is 0 or 1, and q is 0 or a positive integer, provided that p and q are not zero simultaneously. More preferably R, $R^1$ and $R^2$ are independently hydrogen, alkyl of 1 to 4 carbon atoms or phenyl, p is 1 and q is 1 or 2.

Particularly useful A and D groups of the structure above can be represented by the following groups I to III while particularly useful B groups can be the divalent analogues of the monovalent groups I to III.

I. 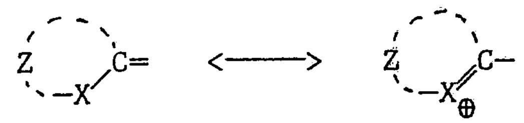 or

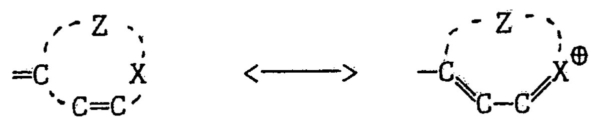

wherein X is oxy, thio or amino($-NR^3-$wherein $R^3$ is hydrogen, substituted or unsubstituted alkyl, preferably of 1 to 10 carbon atoms, for example, methyl, ethyl, isopropyl, chlorobutyl or benzyl, substituted or unsubstituted aryl, preferably of 6 to 12 carbon atoms, for example, phenyl, naphthyl or chlorophenyl), and Z represents the carbon or hetero atoms needed to complete a substituted or unsubstituted 5- to 10-membered ring (including a fused ring group).

II. 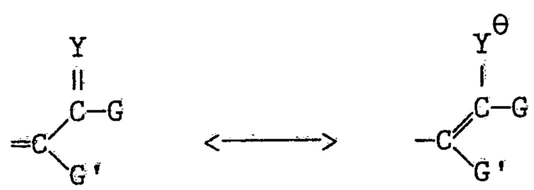

wherein Y is oxo, thioxo, G is hydroxy, substituted or unsubstituted alkyl (preferably of 1 to 10 carbon atoms for example, methyl, ethyl, isopropyl, chloromethyl or benzyl), substituted or unsubstituted aryl (preferably of 6 to 12 carbon atoms, for example, phenyl, naphthyl, xylyl or chlorophenyl), amino ($-NR^4R^5$ wherein $R^4$ and $R^5$ are independently as defined for $R^3$ above), substituted or unsubstituted alkoxy (preferably of 1 to 10 carbon atoms, for example, methoxy, ethoxy or propoxy) or aryloxy preferably of 6 to 10 carbon atoms, such as phenoxy, and G′ is any of the groups listed for G and in addition can be cyano, arylsulfonyl or a group represented by

$-\overset{\overset{O}{\|}}{C}-G$, or G′ taken together with G can represent the atoms necessary to complete a carbocyclic or heterocyclic ring, such as 2,4-oxazolidinone, 2,4-thiazolidinone, rhodamine, hydantoin, 2-pyrazolin-5-one,

1,3-indanedione or barbituric acid.

III. 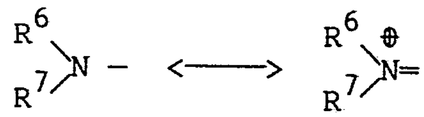

wherein $R^6$ and $R^7$ are independently hydrogen or defined as $R^3$ above, or together represent the atoms needed to complete a substituted or unsubstituted 5- to 10- membered heterocyclic ring (including fused ring groups), for example, pyrrolidone, 3-pyrroline, 4-methylpiperazine, morpholine, 1,2,3,4-tetrahydroquinoline or indoline

IV. Group A can also be derived from a styrene molecule containing substituents in the ortho or para positions, for example, alkyl (preferably of 1 to 12 carbon atoms, for example, methyl, ethyl, hexyl or decyl), alkoxy (preferably of 1 to 12 carbon atoms, for example methoxy or ethoxy), or amino ($-NR^8R^9$ wherein $R^8$ and $R^9$ are independently hydrogen or alkyl of 1 to 6 carbon atoms as defined above).

Representative useful methine dyes are those listed in Table I below with Dye 1 being preferred. Other useful dyes are described in Research Disclosure, publication 22534, January, 1983, pp. 25-28 and references mentioned therein and in U.S. Reissue Patent 30,267. The oxidation potentials listed in Table I below were measured according to the procedure of the Bond et al reference noted above.

TABLE I

| Dye | $\lambda_{max}$(nm) | Oxidation Potential (volts) | Structure |
| --- | --- | --- | --- |
| 1 | 710 | +0.554 | 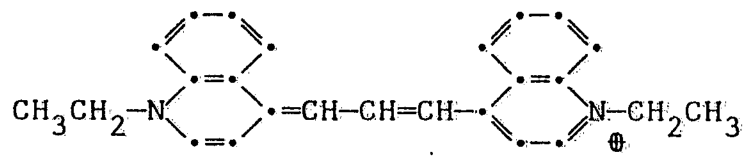 $ClO_4^-$ , |
| 2 | 653 | +0.630 | 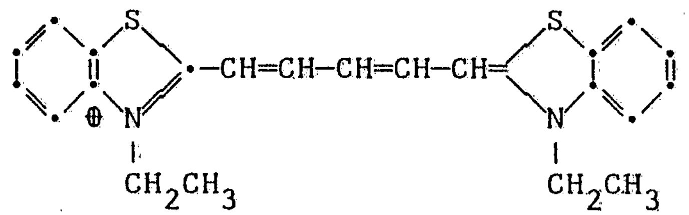 p-toluenesulfonate, |
| 3 | 605 | +0.764 | 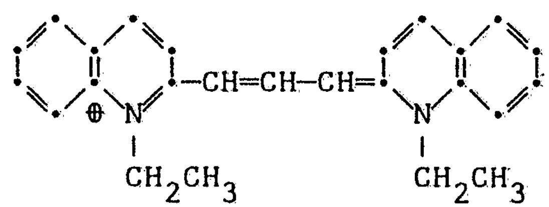 $Cl^-$ , |

TABLE I Cont'd.

| Dye | $\lambda_{max}$(nm) | Oxidation Potential (volts) | Structure |
|---|---|---|---|
| 4 | 543 | +0.400 | 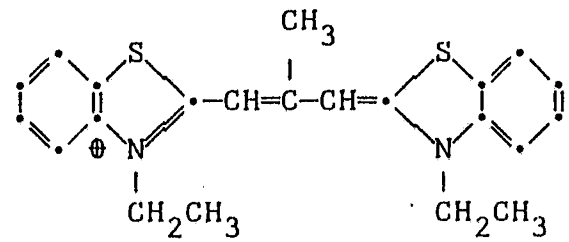 p-toluenesulfonate, |
| 5 | 485 | +1.020 | 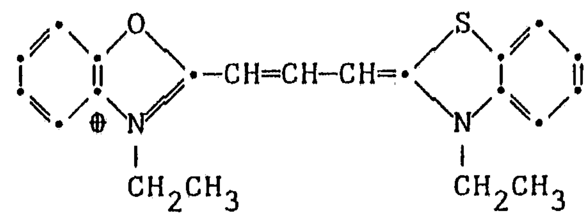 p-toluenesulfonate, and |
| 6 | 615 | +0.280 | 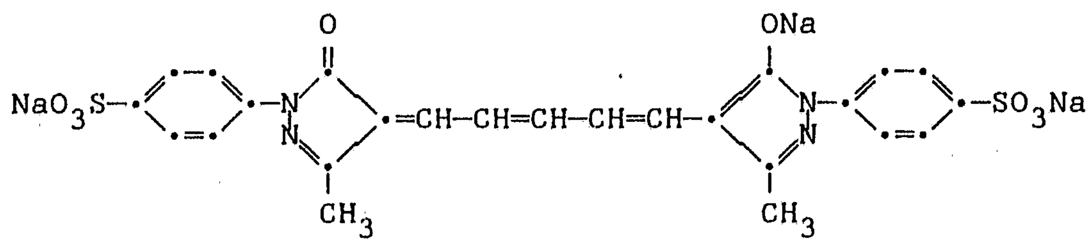 |

Upon oxidation by hydrogen peroxide in the presence of a substance having peroxidative activity (for example, peroxidase), the methine dyes described above are either bleached, (that is, lose part or all of the

dye density), or undergo a shift in absorbance while substantially maintaining dye density. In either case, the change is detectable either visually or with suitable spectrophotometric equipment. The time at which the change is detectable will vary with the dye and whether the assay is performed in solution or in a dry element, but generally, it occurs before 30 minutes. In preferred embodiments, a change is detectable in about 5 minutes.

The methine dyes useful in the practice of this invention can be prepared using readily available starting materials and known analytical procedures.

In one embodiment, the analytical composition of this invention includes a methine dye as described above, and a substance having peroxidative activity. Such substances are also known as peroxidative substances and are capable of catalyzing the oxidation of another substance by means of hydrogen peroxide or other peroxides. Such substances include natural and synthetic peroxidases and cytochromes, hemin, forms of hemoglobin, hematin, iron sulfocyanate, iron tannate, chromic salts and the like. Peroxidase is a particularly useful peroxidative substance.

This analytical composition can also be buffered to a desired pH. Useful buffers include carbonates, borates, phosphates, glutarates, the tris materials, for example, tris(hydroxymethyl)amino-methane and others known in the art as described by Good et al in Biochem., 5, 467 (1966) and Anal. Biochem., 104, 300 (1980).

It may be desirable to include an electron transfer agent in the analytical composition in order to promote more rapid oxidation of the dye. Suitable electron transfer agents include phenazine methosulfate, phenazine ethosulfate, 4′-hydroxyacetanilide, p,p′-biphenol and other phenols, and benzo- and naphthoquinones.

The analytical composition of this invention can be prepared for use in a solution assay by mixing the peroxidative substance (generally in an aqueous solution) with the methine dye in a suitable solvent. The details of preparing a representative composition are given in Example 1 below.

When the analytical composition of this invention is used in a solution assay, generally the methine dye is present in a concentration that depends upon the extinction coefficient of the dye, and generally at least $10^{-6}$ molar. Similarly, the peroxidative substance is present in an amount sufficient to catalyze the oxidation of the methine dye. For example, peroxidase is present in an amount of at least $10^{-14}$ molar, and preferably from $10^{-12}$ to $10^{-8}$ molar. The amounts of optional components (for example, buffer, surfactant or electron transfer agent) and of the interactive composition (described below) are within the skill of an ordinary worker in the art.

In another embodiment, the analytical composition of this invention can be used to determine an analyte which is capable of producing hydrogen peroxide (that is, a substance which can participate in a reaction or series of reactions which produce hydrogen peroxide) in an aqueous liquid. Such analytical compositions comprise an interactive composition which produces hydrogen peroxide upon interaction with the analyte. Analytes which can be determined in this manner include glucose, triglycerides, uric acid, cholesterol, galactose, amino acids, creatine kinase, and others known to one skilled in the clinical chemistry art. For example, an interactive composition for determining uric acid includes uricase, and an interactive composition for determining cholesterol includes cholesterol oxidase and cholesterol ester hydrolase. Further, a representative interactive composition for determining creatine kinase includes glycerol kinase, adenosine triphosphate and α-glycerophosphate oxidase. Other interactive compositions can be devised for a given analyte by those skilled in the art.

The analytical composition of this invention can also be used to determine oxidoreductases or Fe(III)-containing compounds, for example, hemin or chelates containing iron, or metalloporphyrins containing various metal atoms.

In still another embodiment, the present invention can be used to determine a peroxidative substance (as defined above). In this embodiment, an analytical composition comprises a methine dye used in conjunction with hydrogen peroxide or an interactive composition which reacts to produce hydrogen peroxide to produce a detectable change in the presence of a peroxidative substance. Such an assay can be useful, for example, in specific binding assays where peroxidase is used as a label for a ligand (for example, drug, hapten, antigen, antibody) to be determined.

The method of this invention is adaptable to both solution and dry assays. In a solution assay for hydrogen peroxide, generally the methine dye, peroxidative substance and any other reagents are physically contacted and mixed with a liquid test sample suspected of containing hydrogen peroxide in a suitable container (for example, a test tube, petri dish, beaker or cuvette). In an assay for a peroxidative substance in a test sample, hydrogen peroxide is similarly mixed with the dye and the test sample. In either case, the resulting solution is incubated for a relatively short time (that is, up to 30 minutes) at a temperature of up to 45°C. The sample is then evaluated by measuring the change in dye density or the shift in dye absorption. This change can then be correlated to the amount of analyte. Such an evaluation can be done visually or with suitable spectrophotometric detection equipment and procedures.

Alternatively, this invention can be utilized with a dry analytical element which can be a simple absorbent carrier material, that is, a thin sheet or self-supporting absorbent or bibulous material, such as filter paper or strips, which contains the methine dye. While the peroxidative substance or hydrogen peroxide can be separately added to the element at the time of the assay, such reagents can also be included in the element. Such elements are known in the art as test strips, diagnostic elements, dip sticks, diagnostic agents and the like.

When employed in dry elements, the compositions of this invention can be incorporated into a suitable carrier material by imbibition, impregnation, coating or other suitable technique. Useful carrier materials are

insoluble and maintain their structural integrity when exposed to water or physiological fluids such as urine or serum. For example, they can be prepared from paper, porous particulate structures, porous polymeric films, cellulose, glass fibers, woven and nonwoven fabrics (synthetic and nonsynthetic) and the like. A useful dry element is made by imbibing a solution of the appropriate composition into the carrier material and drying. Useful materials and procedures for making such elements are well known in the art as exemplified in U.S. Patents 3,092,465, 3,802,842, 3,915,647, 3,917,453, 3,936,357, 4,248,829, 4,255,384, and 4,270,920, and U.K. Patent 2,052,057.

Preferably, the dry analytical element of this invention has at least one porous spreading zone. This zone can be a self-supporting carrier material (that is, composed of a material rigid enough to maintain its integrity), but generally it is carried on a separate support. Such a support can be any suitable dimensionally stable, and preferably, nonporous and transparent (that is, radiation transmissive) material which transmits electromagnetic radiation of a wavelength between 200 and 900 nm. A support of choice for a particular element should be compatible with the intended mode of detection (reflection or transmission spectroscopy). Useful support materials include paper, metal foils, polystyrene, polyesters, polycarbonates and cellulose esters.

The porous spreading zone can be prepared from any suitable fibrous or non-fibrous material or mixtures of either or both, as described, for example, in U. S. Patents 4,292,272, 3,992,158, 4,258,001 and 4,430,436, and Japanese Patent Publication 57(1982)-101760.

The elements can have more than one zone, for example, one or more reagent zones, spreading zones, registration zones, mordant zones, radiation-blocking or filter zones, subbing zones, barrier zones or buffer zones. The zones are generally in fluid contact with each other meaning that fluids, reagents and reaction products can pass between superposed regions of adjacent zones. Preferably, the zones are separately coated layers, although the zones can be separate areas in a single layer. Besides the references noted above, suitable element formats and components are described, for example, in U. S. Patents 4,042,335, 4,132,528, and 4,144,306.

The components of the compositions of this invention can be incorporated in any of the zones of the elements that would be suitable for the particular analysis. The location of individual components is within the skill of a worker in the clinical chemistry art.

In the elements of this invention, the amount of the methine dye can be varied widely, and depends on the extinction coefficient of the dye, but it is generally present in a coverage of at least 0.001, and preferably from 0.01 to 0.2 g/m$^2$. The peroxidative substance is present at a coverage readily determined by a worker skilled in the art. For peroxidase, for example, the coverage is at least 2000, and preferably from 5000 to 50,000, I.U./m$^2$. When the peroxidative substance is the analyte of interest, an interactive composition may be added to or incorporated into the element which composition is capable of generating the peroxide in the amount desired. Alternatively, hydrogen peroxide can be added to the element or, in some instances, be incorporated into the element. A variety of other desirable, but optional reagents and addenda can be present in the element in amounts known to one skilled in the art. Such materials include surfactants, buffers, binders, pigments, activators and reagents for the interactive compositions.

One embodiment of this invention is a multilayer dry analytical element for determining an analyte. This element comprises a support having thereon, in order and in fluid contact, a hydrophilic registration layer containing a hydrophilic binder material, such as gelatin or polyacrylamide, and a spreading/reagent layer. The element also contains: 1) an interactive composition which produces hydrogen peroxide upon interaction with the analyte, 2) a substance having peroxidative activity, and 3) a methine dye as described herein in the same or different layers.

In another embodiment, the present invention is used to determine a specific binding ligand in competitive binding assays, e.g. immunoassays, using a dry analytical element. In this case, peroxidase is used as the ligand label, and the methine dye is used to determine the amount of analyte by determining either bound or unbound label.

A variety of different elements, depending on the method of assay, can be prepared in accordance with the present invention. Elements can be configured in a variety of forms, including elongated tapes of any desired width, sheets, slides or chips.

The assay of this invention can be manual or automated. In general, in using the dry elements, hydrogen peroxide or analyte determination is made by taking the element form a supply roll, chip packet or other source and physically contacting it with a sample (for example, 1 to 200 $\mu\ell$) of the liquid to be tested so that the test sample and reagents mix within the element. Such contact can be accomplished in any suitable manner, for example, by dipping or immersing the element into the sample or, preferably, by spotting the element by hand or machine with a drop of the sample with a suitable dispensing means.

After sample application, the element can be exposed to conditioning, such as incubation, heating or the like, that may be desirable to quicken or otherwise facilitate obtaining any test result.

Determination of hydrogen peroxide or an analyte is achieved when the methine dye is oxidized producing a detectable change in dye density or change in dye absorption. This change can be detected visually or with suitable spectrophotometric means and procedures.

As used in the context of this disclosure and the claims, I.U. represents the International Unit for enzyme activity defined as one I.U. being the amount of enzyme activity required to catalyze the conversion of 1 μmole of substrate per minute under standard pH and temperature conditions for the enzyme.

Example 1: Solution Assay for Hydrogen Peroxide

This example demonstrates the use of Dye 1 identified in Table I above to detect various concentrations of hydrogen peroxide in a solution assay.

Solutions were prepared with Dye 1 ($7.23 \times 10^{-6}$ molar) and peroxidase ($6.3 \times 10^{-9}$ molar) in sodium phosphate buffer (0.2 molar, pH 6.5). Various concentrations of hydrogen peroxide were added. After 5 minutes incubation at 25° C, the optical density (OD) resulting from methine dye oxidation was determined at 710 nm for each solution as well as a Control solution lacking hydrogen peroxide. The results, shown in Table II below, indicate that various concentrations of hydrogen peroxide can be readily detected in a solution assay according to the present invention.

TABLE II

| $H_2O_2$ Concentration (molar) | Peroxidase Concentration (molar) | OD at 710 nm, After 5 min. at 25°C |
|---|---|---|
| 0 (Control) | $6.3 \times 10^{-9}$ | 1.070 |
| $10^{-8}$ | $6.3 \times 10^{-9}$ | 1.038 |
| $10^{-7}$ | $6.3 \times 10^{-9}$ | 1.011 |
| $10^{-6}$ | $6.3 \times 10^{-9}$ | 0.624 |
| $10^{-5}$ | $6.3 \times 10^{-9}$ | 0.003 |

Example 2: Solution Assay for Peroxidase

This example demonstrates the use of Dye 1 to detect low levels of peroxidase in a solution assay.

Solutions were prepared from Dye 1 ($5.2 \times 10^{-6}$ molar) and hydrogen peroxide ($1 \times 10^{-4}$ molar) in sodium phosphate buffer (0.2 molar, pH 6.5) containing 0.1% TRITON X-165 surfactant. Various concentrations of peroxidase were added and the change in optical density ($\Delta OD$) at 710 nm resulting from methine dye oxidation was determined after incubation at 37° C for 5 minutes. Control A (lacking hydrogen peroxide and peroxidase) and Control B (lacking peroxidase) were similarly tested. The results, shown in Table III below,

indicate that this invention can detect peroxidase at low concentrations.

TABLE III

| Peroxidase Concentration (molar) | ΔOD at 710 nm, After 5 min. at 37°C |
|---|---|
| 0 (Control A) | 0.022 |
| 0 (Control B) | 0.023 |
| $8.8 \times 10^{-12}$ | 0.193 |
| $8.8 \times 10^{-13}$ | 0.043 |

Example 3: Dry Assay for α-Glycerophosphate

This example demonstrates the use of this invention to determine an analyte, α-glycerophosphate, which is capable of reacting to produce hydrogen peroxide, in a dry analytical element.

A dry analytical element having the following format and components was prepared:

ELEMENT FORMAT I

| | | |
|---|---|---|
| Spreading Layer | Barium sulfate | 108 $g/m^2$ |
| | Cellulose acetate | 9 $g/m^2$ |
| | ESTANE resin | 1 $g/m^2$ |
| Registration Layer | Gelatin (hardened) | 6.5 $g/m^2$ |
| | TES buffer | 3 $g/m^2$ |
| | TRITON X-100 surfactant | 1.1 $g/m^2$ |
| | Dye 3 (from Table I above) | 0.05 $g/m^2$ |
| | a-Glycerophosphate-oxidase | 3200 I.U./$m^2$ |
| | Peroxidase | 20,000 I.U./$m^2$ |
| | Poly(ethylene terephthalate) Support | |

A series of solutions containing the analyte were prepared and individually applied to the spreading layer of the element. After incubation of the elements at 37°C for 6 minutes, the reflection densities ($D_R$) were determined at 600 nm. The results, shown in Table IV below, indicate that various levels of a peroxide-producing analyte can be determined with an element of the present invention.

TABLE IV

| Analyte Concentration (mmolar) | $D_R$ at 600 nm, After 5 min. at 37°C |
|---|---|
| 0 (Control) | 1.327 |
| 0.05 | 1.274 |
| 0.10 | 1.249 |
| 0.20 | 1.105 |
| 0.40 | 0.899 |
| 0.60 | 0.745 |

Example 4: Dry Assay for Hydrogen Peroxide

This example demonstrates the use of a dry analytical element to determine hydrogen peroxide according to the present invention.

A dry analytical element having the following format and components was prepared:

ELEMENT FORMAT II

| | | |
|---|---|---|
| Spreading Layer | Barium sulfate | 108 $g/m^2$ |
| | Cellulose acetate | 9 $g/m^2$ |
| | ESTANE resin | 1 $g/m^2$ |
| Subbing Layer | Polyisopropyl-acrylamide | 0.4 $g/m^2$ |
| Registration Layer | Gelatin (hardened) | 11 $g/m^2$ |
| | Dye 1 (from Table I) | 0.025 $g/m^2$ |
| | Peroxidase | 20,000 $I.U./m^2$ |
| | Poly(ethylene terephthalate) Support | |

Solutions 1 and 2 containing hydrogen peroxide ($10^{-4}$ and $10^{-5}$ molar, respectively) in sodium phosphate buffer (pH 6.5) and a Control (lacking hydrogen peroxide) were applied to individual elements. The elements were incubated at 37°C for 5.5 minutes and the rate of dye density change ($D_R$/minute) resulting from methine dye oxidation was measured at 710 nm. Four replicates were made for each solution. The results (average of the replicates) are illustrated in the FIGURE.

**Claims**

1. A composition for the determination of hydrogen peroxide or an analyte which is capable of reacting to produce hydrogen peroxide comprising a peroxidative substance, the composition characterized in that it comprises a methine dye capable of being oxidized by hydrogen peroxide in the presence of the

peroxidative substance.

2. An analytical element for the determination of hydrogen peroxide or an analyte which is capable of reacting to produce hydrogen peroxide comprising an absorbent carrier material and containing a peroxidative substance, the element characterized in that it contains a methine dye capable of being oxidized by hydrogen peroxide in the presence of the peroxidative substance.

3. The invention as claimed in either of claims 1 and 2 further comprising an interactive composition for an analyte which reacts with the analyte to produce hydrogen peroxide.

4. A method for the determination of hydrogen peroxide or an analyte which will react to produce hydrogen peroxide comprising the steps of:

A. in the presence of a peroxidative substance, contacting a sample of a liquid suspected of containing hydrogen peroxide or an analyte which will react to produce hydrogen peroxide with a methine dye capable of being oxidized by hydrogen peroxide to produce a detectable change, and

B. determining the detectable change resulting from the presence of hydrogen peroxide or the analyte.

5. The method as claimed in claim 4 for the determination of the analyte carried out in the presence of an interactive composition for the analyte which reacts to produce hydrogen peroxide.

6. A method for the determination of a peroxidative substance comprising the steps of:

A. in the presence of hydrogen peroxide or an interactive composition which is capable of reacting to produce hydrogen peroxide, contacting a sample of a liquid suspected of containing a peroxidative substance with a methine dye capable of being oxidized by hydrogen peroxide in the presence of the peroxidative substance to produce a detectable change, and

B. determining the detectable change resulting from the presence of the peroxidative substance.

7. The invention as claimed in any of claims 1 to 6 wherein the methine dye is represented by the structure

(A-METH-B $)_{m}($ METH-D$)_{n}$

wherein A, B and D are independently substituted or unsubstituted heterocyclic, carbocyclic, acyclic, aromatic or amino groups, m is 1, n is 0 or 1, and METH is a substituted or unsubstituted methine linkage.

8. The invention as claimed in claim 7 wherein METH is represented by the structure:

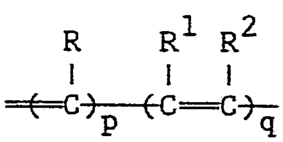

wherein R, $R^1$ and $R^2$ are independently hydrogen, substituted or unsubstituted alkyl, substituted or unsubstituted aryl, or any two of R, $R^1$ and $R^2$ can represent the atoms necessary to complete an alkylene or heterocyclic bridge, p is 0 or 1, and q is 0 or a positive integer, provided that p and q are not zero simultaneously.

9. The invention as claimed in any of claims 1 to 8 wherein the methine dye has an oxidation potential of from 0.20 to 1.5 volts.

10. The invention as claimed in any of claims 1 to 9 wherein the methine dye is selected from the group of

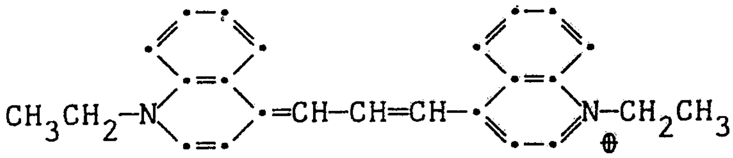

$ClO_4^-$ ,

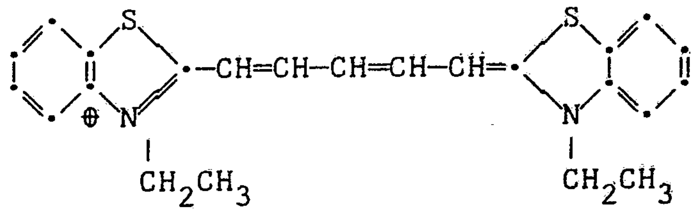

p-toluenesulfonate

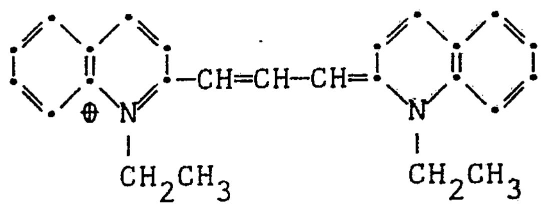

$Cl^-$ ,

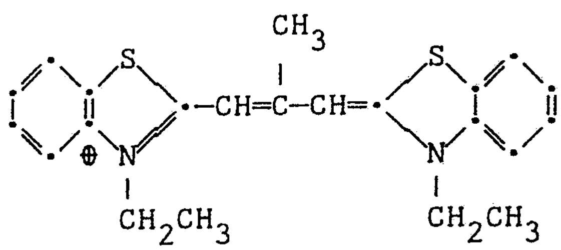

p-toluenesulfonate,

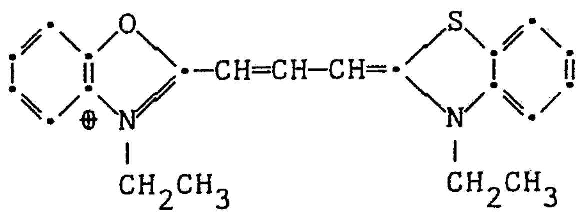

p-toluenesulfonate, and

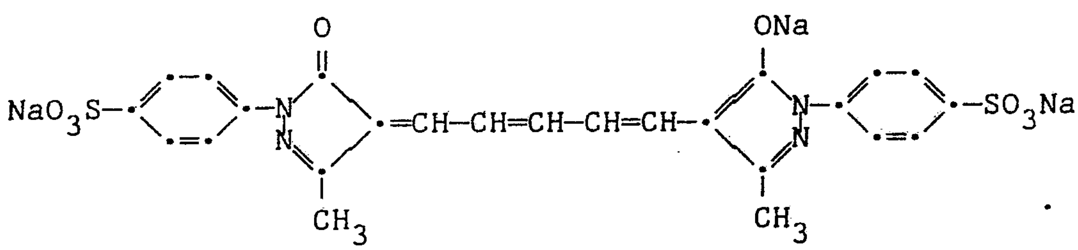

.

11. The invention as claimed in any of claims 1 to 10 wherein the peroxidative substance is peroxidase.

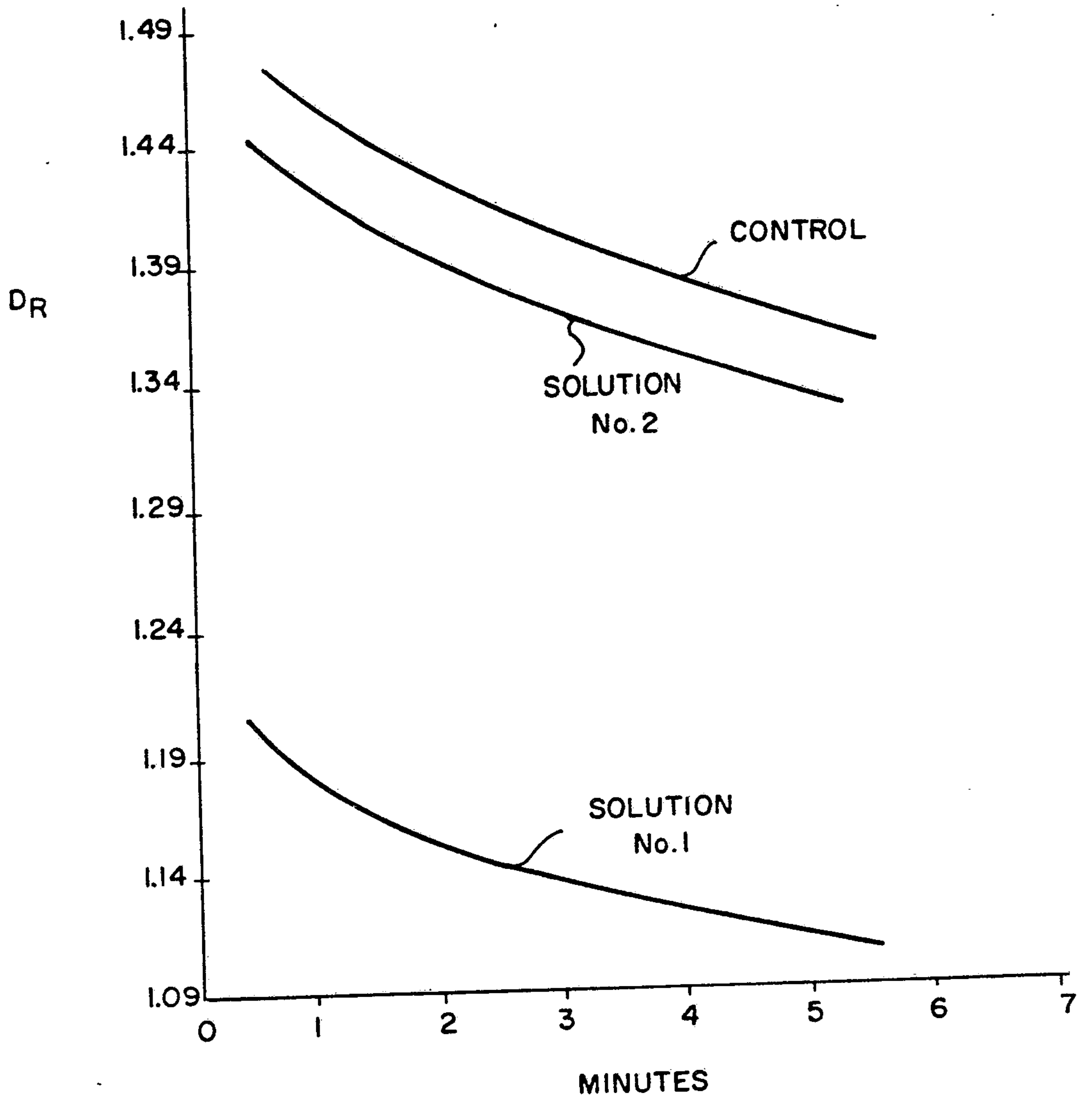
1.49
1.44
1.39
1.34
1.29
1.24
1.19
1.14
1.09
$D_R$
CONTROL
SOLUTION
No. 2
SOLUTION
No. 1
0
1
2
3
4
5
6
7
MINUTES

FIG. 1

European Patent Office

# EUROPEAN SEARCH REPORT

Application number

EP 87 30 4683

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GB-A- 960 167 (MILES LABORATORIES) * complete * | 1-9,11 | C 12 Q 1/28<br>G 01 N 33/52<br>C 09 B 23/00 |
| X | GB-A-1 068 533 (MILES LABORATORIES) * complete * | 1-9,11 | |
| A | EP-A-0 128 521 (EASTMAN KODAK CO.) * page 3, line 34 - page 6, line 23; pages 10-14, formulae; pages 17-33, examples * | 1,10 | |
| A | US-A-4 232 121 (P.B. GILMAN et al.) * columns 3-10, examples * | 10 | **TECHNICAL FIELDS SEARCHED (Int. Cl.4)** |
| A | US-A-2 525 520 (L.G.S. BROOKER et al.) * complete * | 10 | G 01 N 33/00<br>C 12 Q 1/00<br>C 09 B 23/00 |
| A | US-A-1 934 659 (L.G.S. BROOKER et al.) * complete * | 10 | |
| A | US-A-2 138 223 (G. WILLIAMS) * claim 1 * | 10 | |
| | --- -/- | | |

The present search report has been drawn up for all claims

| Place of search | Date of completion of the search | Examiner |
|---|---|---|
| BERLIN | 31-07-1987 | GREEN C.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application number

EP 87 30 4683



## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 86, no. 19, 9th May 1977, page 244, column 1, abstract no. 135920w, Columbus, Ohio, US; ANON "Improved multilayer elment", & RES. DISCL. 1977, 155, 55-62<br>----- | 1-9,11 | |

TECHNICAL FIELDS SEARCHED (Int. Cl.4)

The present search report has been drawn up for all claims

| Place of search | Date of completion of the search | Examiner |
|---|---|---|
| BERLIN | 31-07-1987 | GREEN C.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82